# EUROPEAN PATENT APPLICATION

(11) **EP 0 985 384 A1**
(43) Date of publication of application: **15.03.2000**
(21) Application number: 99117487.1
(22) Date of filing: 10.09.1999
(51) Int. Cl.: A61F 2/30, A61F 2/36, C21D 7/06, B24C 1/10

(54) **Method for improving strenght of prosthetic component**

(30) Priority: 10.09.1998 US 99707
(71) Applicant: Buechel-Pappas Trust, South Orange, New Jersey 07079 (US)
(72) Inventor: Pappas, Michael J., Caldwell, New Jersey 07006 (US); Buechel, Frederick F., South Orange, New Jersey 07079 (US)
(74) Representative: Müller-Boré & Partner Patentanwälte

(57) **Abstract**

A prosthesis (10) is provided with enhanced resistance to fatigue related failures. The prosthesis (10) is formed from a metal material and has at least one surface region subject to shot peening. The shot peening creates an array of round bottom shallow depressions that impart to the surface a built-in compressive stress. The shot peening is particularly effective for the tapered end of a modular stem (24) to both avoid fatigue related failures and to achieve frictional engagement with a stem extension.

## Description

### BACKGROUND OF THE INVENTION

1. Field of the Invention. Fatigue in metals under load occurs primarily as a result of fluctuating tensile stresses on the surface of the part. This pulling of the surface, if great enough, will produce a crack (this is called crack initiation). Severe stress concentrations are present at the tip of the crack. Thus, as the stress fluctuations continue, the crack will grow (this is called crack propagation) until the part is weakened sufficiently so the applied load produces catastrophic failure of the part.

The fatigue strength of a part is dependent on the condition of its surface, A rough surface contains many areas of localized stress concentration providing excellent points for crack initiation. Built in surface tensile stresses also reduce the strength of the part since the added stress from the stress resulting from the applied load, and thus the applied load required to produce crack initiation and propagation, are lessened by this initial, built in, surfaces stress.

Stress concentrations can also result from variations in the shape and stiffness of the part and in regions of the applied load.

The reaction of a metal to stress concentration is dependent on its "notch sensitivity". Materials with greater sensitivity are more affected by stress concentrations than those with lower sensitivity. These terms and effects are well understood in the aerospace or other industries where lightweight and high strength is important. They are not well understood in the orthopedics industry.

A problem associated with fatigue became apparent in the development of connections for modular tapered end stems of prosthetic devices, These stems failed, at the taper connection, during fatigue at relatively low loading due to the notch sensitivity of the titanium alloy used. The modular stem is polished along its length, but the connection end must be rough to obtain good connection strength since friction is a large part of the connection forces. Changes to the method of machining or grinding the tapered surface were able to reduce the local stress concentrations associated with a rough surface and hence produced some increase in strength. However, these improvements were insufficient.

### SUMMARY OF THE INVENTION

The subject invention is directed to a method of manufacturing a prosthetic component, and particularly the tapered end of a modular stem of a prosthetic component, by shot peening the taper. Shot peening works primarily by introducing a built in compressive stress into the surface. This effect increases the fatigue strength of the part in the reverse of the manner in which built in tensile stress reduces the fatigue. Where an initial compressive stress is present, tensile stress resulting from the applied load is reduced by the amount of the built in stress, thus reducing the peak tensile stress resulting from the applied load. This of course means that the load needed to break the part after a given number of cycles is increased.

Shot peening is achieved by firing small balls at the surface with sufficient force to reduce surface stress concentrations as compared to the surface stress concentrations that would be present on a tapered stem that had been roughened to produce an array of sharp-bottom grooves or pits. The shot peening of the taper will produce a roughened surface defined by an array of round-bottom shallow depressions. The round-bottom shallow depressions on the taper provide good connection strength with the modular stem extension due to friction. However, the built in compressive stress achieved by the shot peening results in a substantial avoidance of fatigue related failure as compared to prior tapered stems with roughening achieved by sharp-bottomed grooves. The prosthesis may be made from a titanium alloy and may have a titanium nitride coating applied after the shot peening.

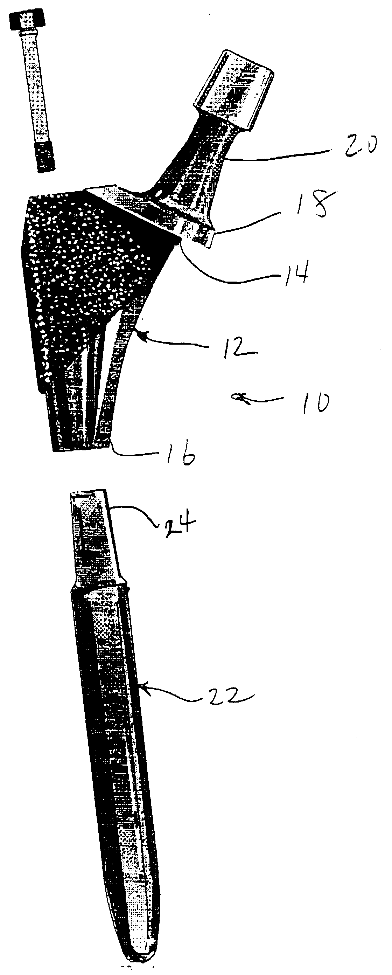

### DESCRIPTION OF THE DRAWING

The Figure is an exploded side elevational view of a modular femoral prosthesis in accordance with the subject invention.

### DETAILED DESCRIPTION OF THE PREFERRED EMBODIMENT

A modular femoral prosthesis in accordance with the subject invention is identified generally by the numeral 10 in the Figure. The modular prosthesis 10 includes a stem 12 having a proximal end 14 and a distal end 16. A collar 18 is disposed at the proximal end 14 of the stem 12 and will be positioned on the resected proximal end of the femur. A neck 20 extends from the collar 18 and may be dimensioned to receive a spherical head to replace the natural head of the femur, The stem 12 is implanted within the intramedulary cavity of the prepared femur.

The required dimensions of the prosthesis 10 will vary from one patient to another. Accordingly, the femoral prosthesis 10 is provided with a stem extension 22. The stem extension 22 enables the stem 12 of the prosthesis 10 to be lengthened and enables the cross-sectional dimensions to be selected in accordance with cross-sectional dimensions of the intramedulary cavity. Thus, the prosthesis 10 illustrated in the Figure may be one of several optional prostheses that can be assembled from a prosthetic system. The system will include several differently dimensioned extensions 22 and may further include several differently dimensioned stems 12. Each of the plurality of differently dimensioned extensions 22 can be mounted to the distal end 16 of the selected stem 12.

The distal end 16 of the stem 12 is configured as a female component for mating with a male portion of the extension 22. More particularly, the proximal end 24 of the extension 22 defines a taper, and the distal end 16 of the stem 12 is provided with a correspondingly configured recess. Portions of the extension 22 distally of the tapered end 24 typically are polished to provide a smooth surface. The smooth surface prevents bone ingrowth, and thereby enables removal of the prosthesis 10 in the event that a revision surgery becomes necessary. Additionally, the avoidance of bone ingrowth substantially prevents stress shielding that could otherwise lead to disuse atrophy near the resected proximal end of the femur. Although a major portion of the extension 22 is smoothly polished, the tapered end 24 preferably has a rough texture in view of the fact that a major portion of the connection forces between the stem 12 and the extension 22 rely on friction. As noted above, a rough surface contains many areas of localized stress concentration which provide excellent points for crack initiation. Stress concentrations at the tapered end 24 also can be partly attributable to the changes in shape, for the reasons explained above. As a result, prostheses of the general type illustrated in the Figure have been known to fail at the tapered connection due to fatigue at relatively low loading due to the notch sensitivity of the titanium alloy used in the prosthesis 10.

To avoid this problem, portions of the stem extension 22 in proximity to the tapered end 24 are treated by shot peening to introduce a built-in compressive stress into the surface of the extension 22 at the tapered end 24. The shot peening increases the fatigue strength in the reverse of the manner in which built-in tensile stress reduces the fatigue. In particular, tensile stress resulting from the applied load is effectively reduced by the amount of the built-in compressive stress achieved by the shot peening. This effectively reduces the peek tensile stress resulting from the applied load. The shot peening is achieved by firing small balls at the surface of the extension 22 in proximity to the tapered end 24. The shot peening produces on the surface of the extension 22 at and near the taper 24 roughened surface defined by an array of round-bottom shallow depressions. The round-bottom shallow depressions on the taper 24 provide good connection strength with the stem 12 due to friction. However, the built-in compressive stress achieved by the shot peening results in a substantial avoidance of fatigue related failure as compared to the above-described prior art extension tapers with roughening achieved by sharp-bottomed grooves. The prosthesis 10 preferably is made from a titanium alloy that is provided with a titanium nitride coating after the shot peening.

While the invention has been described with respect to a preferred embodiment, it is apparent that various changes can be made without departing from the scope of the invention as defined by the appended claims. In particular, the shot peening described herein may be applied to other regions of prosthetic components that could be subjected to fatigue related failure, such as shoulder prostheses with humeral components that have modular stems.

## Claims

1. A method for producing a prosthetic component, said method comprising the steps of:
forming a prosthetic component into a selected shape; and
subjecting at least portions of the formed prosthetic component to shot peening by directing a plurality of small balls to at least selected surface regions of the prosthetic component for introducing a built-in compressive stress into said surface regions.

2. A method of claim 1, wherein the shot peening is carried out with sufficient force to form in said surface region an array of round-bottom shallow pits.

3. The method of claim 2, wherein the prosthetic component is formed from a titanium alloy.

4. The method of claim 3, further comprising the step of applying a titanium nitride coating to the prosthetic component after the shot peening.

5. The method of claim 1, wherein the prosthetic component includes a tapered stem for mating with a modular prosthetic extension, said shot peening being carried out on the tapered stem of the prosthetic component.

6. A prosthesis formed from a metallic component and having an outer surface, at least one region of said outer surface having an array of round-bottom depressions formed thereon by shot peening the surface region for imparting a built-in compressive stress to the surface region.

7. The prosthesis of claim 6, wherein the metal of the prosthesis is a titanium alloy.

8. The prosthesis of claim 7, wherein the surface has a coating of titanium nitride applied over the array of round-bottom shallow depressions.

9. The prosthesis of claim 8, wherein the prosthesis is a femoral stem (12) prosthesis having an extension (22) with a tapered end configured for frictional engagement with a recess in the femoral stem, the shot-peened round-bottom shallow depressions being provided on the tapered end of the extension.
